# EUROPEAN PATENT APPLICATION

(11) **EP 4 385 395 A1**
(43) Date of publication of application: **19.06.2024**
(21) Application number: 23154502.1
(22) Date of filing: 01.02.2023
(51) Int. Cl.: A61B 5/00

(54) **SLEEP APNEA MONITORING PATCH WITH SURFACE ELECTROMYOGRAPHY (EMG) SIGNAL CAPTURE AND METHOD OF OPERATION THEREOF**

(30) Priority: 13.12.2022 US 202263432092 P
(71) Applicant: Koninklijke Philips N.V., 5656 AG Eindhoven (NL)
(72) Inventor: DE GRAAF, Pascal, 5656AG Eindhoven (NL); DEKKER, Marian, Eindhoven (NL)
(74) Representative: Philips Intellectual Property & Standards

(57) **Abstract**

A mobile electromyography (EMG) device (100), including: a control unit (102) having at least one controller (120) to determine a Neural Respiratory Drive (NRD) measurement, a body (117) having opposed sides (130, 136), an optical sensor (146) extending from one of the opposed sides (130, 136), and a patch (101) having a carrier (165) with a cavity (164) to receive at least part of the body (117), and an opening (145) extending through the patch (101) and configured to receive at least a portion of the optical sensor (146), the patch (101) further having an adhesive backed substrate (124) to releasably couple to the skin of a user, and a plurality of sensors (114) situated apart from each other to sense surface EMG signals from the skin of the user; and a coupler (167) to releasably couple the body (117) of the control unit (102) to the patch (101).

## Description

### FIELD OF THE INVENTION

The present system relates to a portable sleep apnea monitoring system for detecting sleep patterns and, more particularly, to a portable sleep apnea system which employs a patch to integrate several measurements and a method of operation thereof.

### BACKGROUND OF THE INVENTION

Diagnosis of sleep-apnea can be performed at home or in a specialized sleep disorder center, where a person under test (hereinafter patient) is expected to spend a night while several parameters are electronically monitored using a study known as nocturnal polysomnography. These tests include monitoring heart, lung and brain activity, breathing patterns, arm and leg movements, and blood oxygen levels while you sleep at a specialized sleep disorder center.

In contrast, home sleep apnea testing typically measures heart rate, blood oxygen level, airflow and breathing patterns of the patient being tested but cannot detect all cases of sleep apnea because they lack many of the typical tests. When the home test does not produce a clear result, the patient is often referred to spend a night at a sleep disorder center. Often, the stress of sleeping in this unfamiliar environment causes insomnia or different sleep behavior, influencing the measurement and diagnosis and sometimes makes it necessary to spend another night to repeat the measurements at great inconvenience and cost. Therefore, a need exists for a more comprehensive home testing procedure that increases the chance of a successful diagnosis.

Further, conventional tests (both at home and at a sleep disorder center) require a multitude of sensors for different measurements. For example, brain activity sensors are attached to the skull of the patient, a belt around the chest of the patient monitors breathing, electrodes on each foot and several on each arm of the patient monitor muscle movement, a nasal canula or mask monitors airflow of the patient and a microphone records patient snoring. These sensors are connected by cables and hoses to a central unit that is usually placed on a night table near a bed upon which the patient is lying to sleep. These cables and hoses can cause a great deal of discomfort to the patient during sleep which can also cause insomnia or different sleep behavior, influencing the measurements and sometimes makes it necessary to repeat the measurements another night at great inconvenience and cost to the patient taking the test. Additionally, these cables require a great deal of setup time, are uncomfortable and can become wrapped around the patient which can awaken the patient as well as cause injury to the patient.

Accordingly, embodiments of the present system provide features and advantages which obviate conventional diagnostic systems and methods. To overcome the aforementioned barriers and detriments as well as others, there is a need for a mobile sleep apnea system that is compact and can detect sleep apnea at home while increasing user comfort, convenience, ease-of-use, as well as provide a sleep apnea detection system to users who would otherwise not perform sleep apnea testing because of the inconvenience of conventional systems. This can result in diagnosis and treatments for individuals who would otherwise not be able to obtain proper diagnosis and treatment.

### SUMMARY OF THE INVENTION

The system(s), device(s), method(s), arrangements(s), interface(s), computer program(s), processes, etc., (hereinafter each of which will be referred to as system, unless the context indicates otherwise), described herein address problems in prior art systems.

In accordance with embodiments of the present system, there is disclosed a mobile electromyography (EMG) device including: a control unit having at least one controller, a body having opposed sides, an optical sensor extending from one of the opposed sides, and a plurality of connectors coupled to the at least one controller; a patch having a carrier with a cavity configured to receive at least part of the body, and an opening extending through the patch and configured to receive at least a portion of the optical sensor, the patch further having an adhesive backed substrate configured to releasably couple to the skin of a user, and a plurality of sensors situated apart from each other and configured to sense surface EMG signals from the skin of the user; and a coupler configured to releasably couple the body of the control unit to the patch, wherein the at least one controller may be configured to receive the sensed surface EMG signals from the plurality of sensors and determine a Neural Respiratory Drive (NRD) measurement based thereon. The at least one controller may analyze the EMG signals from the plurality of sensors to determine heart rhythm and body movement signals. The at least one controller may be configured to determine whether apnea exists based upon the NRD measurement and the heart rhythm and body movement signals.

In accordance with embodiments, the at least one controller may be further configured to transmit the NRD measurement and the heart rhythm and body movement signals to a mobile station (MS) for further analysis. The coupler may include at least one of a hook-and-loop fastener and a snap-fit fastener. The body may include an accelerometer situated in a cavity thereof and configured to detect acceleration of the body and output a corresponding signal. The at least one controller may be configured to receive the output signal of the accelerometer and form corresponding acceleration information. The at least one controller may be configured to process the acceleration information to resolve movement artefacts in the EMG signals. The at least one controller may be configured to receive an output signal from the optical sensor and determine peripheral oxygen saturation (SpO2) based thereon.

Further, in accordance with embodiments of the present system, there is disclosed a mobile electromyography (EMG) device including: a control unit having at least one controller, a body having opposed sides, an optical sensor extending from one of the opposed sides, and a plurality of connectors coupled to the at least one controller; a carrier with a cavity configured to receive at least part of the body, and an opening extending through the carrier and configured to receive at least a portion of the optical sensor; a patch having an adhesive backed substrate configured to releasably couple to the skin of a user, and a plurality of EMG sensors situated apart from each other and configured to sense surface electromyography (sEMG) signals from the skin of the user; a coupler configured to releasably couple the body of the control unit to the carrier and being coupled to the patch; and a flexible strip coupled to the carrier and configured to couple to the user, wherein the at least one controller may be configured to receive the sEMG signals from the plurality of sensors and determine a Neural Respiratory Drive (NRD) measurement based thereon. The at least one controller may be further configured to analyze the EMG signals from the plurality of sensors to determine heart rhythm and body movement signals. The at least one controller may be further configured to determine whether apnea exists based upon the NRD measurement and the heart rhythm and body movement signals.

The at least one controller may be further configured to transmit the NRD measurement and the heart rhythm and body movement signals to a mobile station (MS) for further analysis. The coupler may include at least one of a hook-and-loop fastener and a snap-fit fastener. The body may further include an accelerometer situated in a cavity thereof and configured to detect acceleration of the body and output a corresponding signal that the at least one controller may process to resolve movement artefacts from the NRD measurement. The at least one controller may be further configured to receive an output signal from the optical sensor and determine peripheral oxygen saturation (SpO2) based thereon. The flexible strip may include an adjustable loop.

In addition, in accordance with embodiments of the present system, there is disclosed a electromyography (EMG) electrode patch including: a carrier with a cavity configured to receive at least part of a body, and an opening extending through the carrier and configured to receive at least a portion of an optical sensor, the carrier further having a plurality of sensors situated apart from each other and configured to sense surface EMG signals from the skin of the user; and a coupler configured to releasably couple the body of a control unit to the carrier. The EMG patch may further include a plurality of contacts with each contact coupled to a corresponding sensor of the plurality of sensors and configured to couple to a corresponding biased contact of a plurality of biased contacts coupled to the body of the control unit. The EMG patch may further include the control unit having at least one controller, the control unit having the body and the optical sensor, the body having opposed sides, the optical sensor extending from one of the opposed sides.

### BRIEF DESCRIPTION OF THE DRAWINGS

It should be expressly understood that the drawings are included for illustrative purposes and do not represent the scope of the present system. It is to be understood that the figures may not be drawn to scale. Further, the relation between objects in a figure may not be to scale and may in fact have a reverse relationship as to size. The figures are intended to bring understanding and clarity to the structure of each object shown and thus, some features may be exaggerated in order to illustrate a specific feature of a structure. In the accompanying drawings, like reference numbers in different drawings may designate identical or similar elements, portions of similar elements and/or elements with similar functionality. The present system is explained in further detail and by way of example, with reference to the accompanying drawings which show features of various exemplary embodiments that may be combinable and/or severable wherein:
FIG. 1 shows an illustrative schematic block diagram of a portion of a system operating in accordance with embodiments of the present system;
FIG. 2 shows an illustrative exploded front side view of a portion of an apnea measuring unit (AMU) in accordance with embodiments of the present system;
FIG. 3 shows an illustrative front side view of a portion of the control unit coupled to the patch of the AMU of FIG. 2 in accordance with embodiments of the present system;
FIG. 4 shows an illustrative top view of a portion of the control unit of FIG. 2 in accordance with embodiments of the present system;
FIG. 5 shows an illustrative bottom view of a portion of the control unit of FIG. 2 in accordance with embodiments of the present system;
FIG. 6 shows an illustrative rear side view of a portion of the control unit of FIG. 2 in accordance with embodiments of the present system;
FIG. 7 shows an illustrative first end view of a portion of the control unit of FIG. 2 in accordance with embodiments of the present system;
FIG. 8 illustratively shows a second end view of a portion of the control unit of FIG. 2 in accordance with embodiments of the present system;
FIG. 9 shows an illustrative top planar view of the patch of the system of FIG. 2 in accordance with embodiments of the present system;
FIG. 10 illustratively shows a top planar end view of a patch of the system of FIG. 2 in accordance with embodiments of the present system;
FIG. 11 shows an illustrative top view of a portion of the AMU with the control unit coupled to the patch in accordance with embodiments of the present system;
FIG. 12 shows an illustrative partially exploded top view of a system with a control unit mounted separately from a patch in accordance with embodiments of the present system;
FIG. 13 shows an illustrative functional flow diagram performed by a process in accordance with embodiments of the present system; and
FIG. 14 shows an illustrative block diagram of a portion of a system in accordance with embodiments of the present system.

### DETAILED DESCRIPTION OF EMBODIMENTS

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well-known devices, circuits, tools, techniques, and methods are omitted so as not to obscure the description of the present system.

The term "and/or," and formatives thereof, should be understood to mean that only one or more of the recited elements may need to be suitably present (e.g., only one recited element is present, two of the recited elements may be present, etc., up to all of the recited elements may be present) in a system in accordance with the claims recitation and in accordance with one or more embodiments of the present system. In the context of the present embodiments, the terms "about", "substantially" and "approximately" denote an interval of accuracy that a person skilled in the art will understand to still ensure the technical effect of the feature in question which in some cases may also denote "within engineering tolerances." For example, the terms may indicate a deviation from the indicated numerical value(s) of ±20 %, ±15 %, ±10 %, ±5 %, ±1 % ±0.5 % or ±0.1 %.

The terms clinician, clinicians, or formatives thereof, may include a professional such as doctor, a clinician, an operator, an expert, a medical specialist, a technician, and/or the like, who may operate and/or review information related to the patient such as system information, and/or data generated by embodiments of the present system and associated data. The terms patient, patients, or formatives thereof, may include patients or other individuals (e.g., subjects, subjects under test, etc.) or other users who may be receiving any type of sleep apnea monitoring using systems, machines, processes, and/or methods operating in accordance with embodiments of the present system.

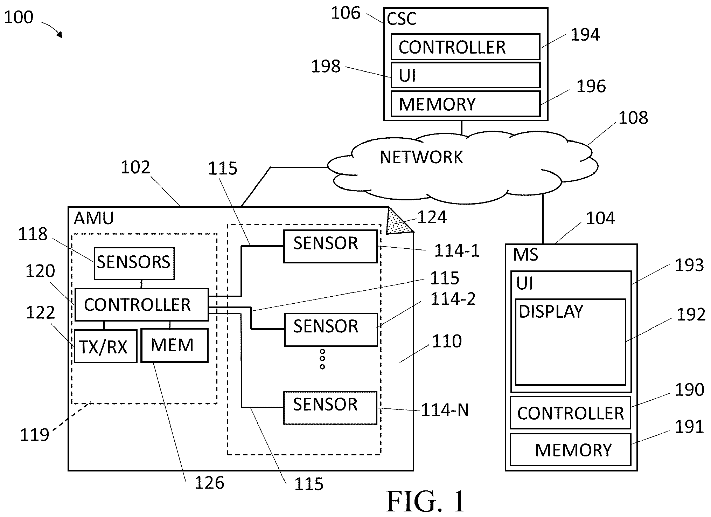

FIG. 1 illustratively shows a schematic block diagram of a portion of a system 100 (hereinafter the system 100) operating in accordance with embodiments of the present system. The system 100 may include one or more of an apnea measuring unit (AMU) 102, a mobile station (MS) 104, a clinical service center (CSC) 106, and a network 108, one or more of which may communicate with the other via any suitable communication method or methods such as wired, wireless, and/or optical communication methods over the network 108. Accordingly, the network 108 may include any suitable network such as an ad-hoc network, a body area network (BAN), a personal area network (e.g., e.g., Bluetooth^{™}, etc.), a Zigbee network, Wi-Fi, a local area network (LAN), a wide area network (WAN), a telephony network, a cellular network (e.g., 5G, etc.), etc. The AMU 102, the MS 104, the CSC 106 and the network 108 may be controlled by at least one controller which may control the overall operation of the system and may include one or more logic devices such as a microprocessor and/or the like, typically coupled to a memory. The controller may control the overall operation of the system 100 and may be distributed to different portions of the system 100 such as distributed to one or more of the AMU 102, the MS 104 and the CSC 106. It should be appreciated that in some embodiments the controller may include digital and/or analog control circuitry. The memory may be any type of device for storing application data as well as other data related to the described operation such as application data, operating parameters, user supplied information, sensor measurements, etc., and/or combinations thereof. The application data, operating parameters, user supplied information, sensor measurements, etc., and/or combinations thereof, may be received by the one or more controllers for configuring (e.g., programming) the controllers to perform operation acts in accordance with the present system. The controller(s) so configured becomes special purpose machines particularly suited for performing in accordance with embodiments of the present system.

The at least one controller may access the memory and may obtain system setting information (SSI) which may include one or more of operating parameters (e.g., scan type, etc.), threshold values, warnings, display settings, user information, patient information such as patient identification (ID), etc. The SSI may be set by the system and/or user and may be updated during use. Accordingly, the system may generate and render an interface with which the patient and/or clinician may, for example, interact to, for example, set, reset, select, and/or adjust one or more settings of the SSI and/or enter information (e.g., ID, results, etc.) which may be stored in the SSI.

The MS 104 may include any suitable mobile station such as a smartphone (e.g., an I-Phone, a Galaxy phone, etc.), personal digital assistant (PDA), a smart watch (e.g., an Apple^{™} Watch^{™}, a Galaxy^{™} watch, etc.), and/or the like, and may include a memory 191, at least one controller 190, a user interface (UI) 193, and a transceiver (e.g., a transmission/reception device) (Tx/Rx) configured to communicate with the network 108. The UI 193 may provide an interface with which a user (e.g., the patient) may interact, such interfaces may include, inter alia, a display 192 such as a touch-screen display, an optical sensor such as a camera, a proximity sensor, a speaker, and/or a microphone. However, other user interface devices are also envisioned. Accordingly, the UI 193 may render information, such as content, etc., on a speaker and/or display for the convenience of the patient, and may receive information entered by the patient such as selections, requests, commands, patient settings, etc., using any suitable input method such as gesture inputs, a touch input, voice input, etc. The voice input may be input via the microphone, if desired.

The memory 191 may store information for use by the system such as the SSI, electromyography signals (EMG), accelerometer information (ACC), peripheral oxygen saturation data (SpO2), system settings, system parameters, operating parameters, patient ID and/or combinations thereof, as will be discussed herein. The patient ID may be employed to identify a patient using the MS 104. In operation, the MS 104 may transmit information such as operating parameters, instructions, requests, commands (e.g., a synchronization command, etc.), to the AMU 102 and may receive information generated by the AMU 102 such as EMG, SpO2, ACC, etc., system parameters (e.g., battery charge), operating parameters, and/or combinations thereof, from the AMU 102 for further processing. Data transfer may occur on a periodic and/or non-periodic intervals as may be set forth in the SSI.
The CSC 106 may include a controller 194, a memory 196 and a UI 198 with which the user may interact. The controller 194 may control the overall operation of the CSC 106 and as such, may receive information, such as the SSI, EMG, ACC, SpO2, results of apnea tests (e.g., apneas detected or not detected, etc.), and/or other information from the AMU 102 and/or MS 104 for further processing. The CSC 106 may further communicate with the AMU 102 and/or the MS 104 to, for example, transmit diagnostic results of the processing (e.g., apnea detected, apnea not detected, etc.), for example, for rendering on a rendering device of the MS 104 for the convenience of the user. For the sake of clarity, only a single MS 104 is swill be discussed unless the context indicates otherwise. However, without limitation one or more other MSs may be employed by the system. For example, a clinician such as a doctor may have an MS which may receive information such as (SSI, EMG, ACC, SpO2, results of tests, etc.) from other portions of the system for further review. The discussions herein related to the MS 104 should be understood to at least optionally apply to any one or more MS.

One or more controllers of the system 100 may employ machine learning and/or artificial intelligence (AI) engines to process information such as the sensor information generated by the system such as the SSI, EMG, ACC and SpO2, results of apnea tests, etc. The machine learning and/or artificial intelligence (AI) engines may detect, for example, the presence or absence of sleep apnea, etc., may store results in a memory of the system and may forward results to the patient or clinician for their convenience. For example, upon processing of the EMG, ACC, and SpO2, if it is detected that the patient has sleep apnea with low SpO2, the system may forward these results to the CSC 106 which may then render these results on the UI 198, such as a display screen, along with the ID of the patient.

The clinician may then contact the patient via one or more of the MS 104 of the patient and/or may contact a doctor or other medical professional (e.g., through their MS), for further analysis and possible action. For example, the clinician may then observe the rendered information and may interact with a user interface generated by the system to enter and/or select information. Further, the clinician may select to contact the patient via the patient's corresponding MS 104 (e.g., as set forth in the SSI) via a selected method (e.g., set by the patient, and/or system) such as via a phone call, a text message, an email, a social media account (e.g., WhatsApp^{™}, Facebook^{™}, etc.), etc. In some embodiments, if certain conditions are detected (e.g., sleep apnea, SpO2 below a threshold, etc.), the CSC 106 may contact the patient via the selected contact method to alert the patient. A clinician may then be informed of the results and may contact the patient (e.g., via the MS 104) and/or a doctor or specialist as may be desired, depending upon system settings. The system may employ different processors for processing one or more of the SSI, EMG, ACC and SpO2, so as to conserve system resources. For example, the SS, EMG, ACC and SpO2 may be forwarded by the AMU 102, in real time or on a periodic or non-periodic intervals, to the MS 104 for analysis. Further analysis may be performed at the CSC 106 which analysis may utilize increased computing power as may be available directly at the AMU 102 and/or the MS 104.

The AMU 102 may include one or more of a controller 120, a Tx/Rx 122, a memory 126, EMG sensors 114-1 through 114-N (generally sensors 114, where N is an integer), one or more sensors 118 and at least one substrate 110. The controller 120 may control the overall operation of the AMU 102.

The substrate 110 may include a flexible substrate that may have an adhesive 124 (e.g., a pressure sensitive adhesive (PSA) configured to releasably couple to the skin of the patient and thereby, be adhered thereto. The adhesive 124 may form a layer that may be located on at least one side of the substrate 110 in a continuous or non-continuous manner, such as at each of the sensors 114, and may firmly adhere to the skin of the patient. The adhesive 124 may include an electrically conductive adhesive layer configured to pass electrical signals (e.g., surface EMG signals) from the skin of the patient to the sensors 114 and may be medical grade. The substrate 110 may have any desired shape and size suitable for its use and it is envisioned that it may be continuous or discontinuous (e.g., formed from one or more sections that may be coupled together). The substrate 110 may be formed from any suitable material or materials such as a medical grade polymer or fabric (e.g., woven, non-woven, etc.) that may include one or more layers superimposed upon each other. The substrate 110 may include instructions printed thereon for instructing a user on proper, placement, orientation, and use. A release liner or liners may be attached to the adhesive 124 to protect the adhesive prior to use and may be removed from the adhesive 124 before use.

The sensors 114 may include EMG sensors configured to sense electrical signals at the skin of the patient and provide these electrical signals to the controller 120 for further processing. Although three sensors 114 are shown for illustration, there may be other numbers of these sensors in accordance with embodiments. In some embodiments, the sensors 114 may be coupled to the controller 120 via leads 115 that may be deposited upon the substrate 110 or may be formed using separate wires. The sensors 114 may be formed integrally with, or separately from, the substrate 110, and may be distributed in a desired pattern relative to the substrate 110 to collect cardiac information from the skin of the patient. In some embodiments, the sensors 114 may be releasably coupled to the controller 120 via a releasable coupler.

In some embodiments, one or more of the controller 120, the Tx/Rx 122, the memory 126, and the sensors 118, may form at least a portion of a control unit (CU) 119 that may be removably coupled to the substrate 110 via a coupler that may further couple one or more of the sensors 114 to the CU 119. Accordingly, the substrate 110, including the sensors 114, may be changed for the convenience of the user (e.g., after use), while the CU 119 may be removable from the substrate 110 (and sensors 114) and may be reused with a new substrate 110. Thus, the substrate 110 including the sensors 114 may be disposable while the CU 119 may be reused with a new substrate 110 including the sensors 114. The CU 119 may include a cavity for housing a power supply such as a capacitor, a battery, and/or the like that may power the CU 119. An optional port may be provided to charge the power supply and/or to communicate with the controller 120 to provide, for example, operating instructions, and/or download data generated, formed, etc., by the AMU 102. The port may include for example, a USB port or the like which may provide for power and/or data transfer. However, in other embodiments, it is envisioned that the CU 119 may be sealed and may be single use or recharged via a wireless charger. Accordingly, the CU 119 may include wireless charging circuits, as desired. The substrate 110 may be placed on the upper chest and/or another location of the patient and may releasably adhere thereto.

The controller 120 may be configured to communicate with the MS 104 using any suitable communication method or methods such as via a Bluetooth^{™} coupling, an RF coupling and/or the like and may synchronize an internal clock with a clock of the MS 104 so that the timing information (e.g., time, day and/or date) that data (e.g., EMG, ACC, and SpO2) is generated, captured, formed, etc., by the controller 120, may be determined and stored with the corresponding information. The synchronization is utilized to assure that the timing information utilized by the AMU and/or MS refer to the same time. It is envisioned that the synchronization routine may be run during operation as may be set forth in the SSI (e.g., every two hours, every two days, etc.) to resynch the clock of the AMU with the clock of the MS. The controller 120 may obtain the SSI from the MS 104 and store this information in the memory 126 for reference at a later time and/or to configure its operation.

During operation, the controller 120 may receive analog signals (e.g., surface EMG) from the sensors 114, amplify (e.g., using an amplifier) and/or condition these analog signals (e.g., using a signal conditioner), convert these signals (e.g., using an analog-to-digital (A/D) converter) to digital signals (e.g., at a desired sampling rate (e.g., as may be set in the SSI)) to form corresponding EMG information which may be further processed to determine breathing effort ( e.g., using Neuro Respiratory Drive (NRD) measurements, such as used by Myotrace^{™}), heart rate, and limb movement information. In accordance with embodiments of the present system, from this, data the respiratory status of the patient may be determined and the process may determine whether any abnormalities such as sleep apnea, low SpO2 (e.g., SpO2 below a threshold value as set in the SSI), abnormal heart rate, or irregular heart rhythm, etc., are present. If it is determined that an abnormality is present (e.g., an irregular heart rhythm, an arrhythmia, low SpO2, etc.), the controller 120 may form alert information indicating the detected abnormality and transmit this alert information to the MS 104 that may, upon determining that the alert information has been received, render information to alert the patient using, for example, one or more rendering devices such as a ringer, a haptic device (e.g., a vibrator, etc.) and may render information of the alert on the display 192 for the convenience of the patient. Settings to control the alert may be set forth in the SSI.

The sensors 118 may include one or more sensors such as an optical sensor (or sensors) to determine (peripheral) oxygen saturation (SpO2), accelerometer to determine acceleration of the control unit 119, and may form corresponding information. In some embodiments, the sensors 118 may further include a touch-sensitive sensor to turn the AMU 102 on and/or off as may be desired. Power savings modes may also be included to turn the AMU 102 on and/or off depending upon whether it is detecting EMG signals to prevent undue battery depletion.

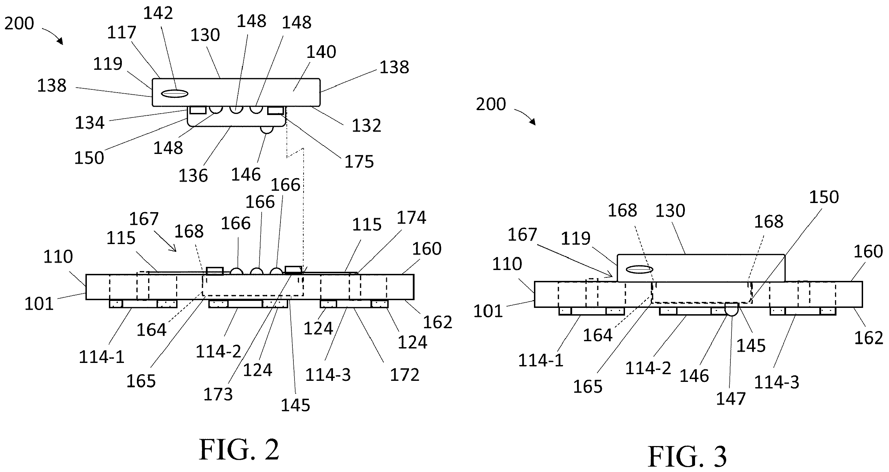

FIG. 2 shows an illustrative exploded front side view of a portion of an AMU 200 in accordance with embodiments of the present system. The AMU 200 may include a control unit 119 releasably couplable to a patch 101. The patch 101 may include the substrate 110, and the sensors 114 coupled to, or formed integrally with, the substrate 110. The sensors 114 may include a hydrogel 172 coupled to electrodes 174 that may be coupled to connectors (e.g., pads) 166 via leads 115 to transfer surface EMG signals from a surface of the skin of the patient to the connectors 166. The leads 115 may be superimposed upon, deposited upon, formed integrally with, or separately from, the substrate 110 and may include a curve to prevent ripping when the substrate 110 may be flexed and/or stretched. The adhesive 124 may be deposited about the hydrogel 172 to assure proper surface conduction between the skin of the patient and the hydrogel 172. The patch 101 may include a carrier 165 including a cavity 164 configured to receive at least a part of the control unit 119 and a coupler 167 configured to releasably couple the control unit 119 to the substrate 110.

The control unit 119 may have a body 117 having an upper surface 130, a lower surface 136, and a flange 132. The upper surface 130 may be situated between end walls 138 and side walls 140. The body 117 may include a cavity configured to include at least a power supply, a controller, a TX/RX, sensor(s), and a memory (e.g., see FIG. 1). The controller may control the overall operation of the control unit 119 and may be coupled to an optional port 142 which may be configured to provide communication and/or power to the CU 119. The controller may also be coupled to connectors 148. That portion of the body 117 that extends between the lower surface 113 and the flange 132 may define a skirt 150 configured to be received in the cavity164 having at least one wall 138 extending thereabout. The bottom wall of the cavity 164 may include an opening 145 configured to receive at least a part of an optic sensor 146 which may include a facetted surface that may engage the skin of the patient.

The connectors 148 may be situated at the flange 132 and may be coupled to the controller. These connectors 148 may be configured to couple to the connectors 166 of the substrate 110 such that surface EMG signals from the sensors 114 may be transmitted to the controller within the CU 119 for further processing in accordance with embodiments of the present system. The connectors 148 may be shaped and sized or otherwise configured to couple to the connectors 166. Further, one or more of the connectors 148, 166 may be spring loaded (e.g., to form a pogo stick type connector or the like). Further, one or more of the connectors 148, 166 may form pads.

The optical sensor 146 may extend from the lower surface 136 and may be configured to extend at least partially through an opening 145 in the cavity 164 when the control unit 119 is coupled to the substrate 110. Accordingly, when the control unit 119 is coupled to the substrate 110 which is coupled to the patient, a faceted end of the optical sensor may contact the skin of the patient such that the optical sensor 146 may obtain specific oxygen signals for determining SpO2 and provide this information to the controller that may be coupled thereto for further processing.

The coupler 167 may include any suitable coupler or couplers, such as an interference type coupler, a snap fit coupler, an adhesive coupler, a band (e.g., an elastic band, etc.) a strap-type coupler, a hook-and-loop type coupler (e.g., Velcro^{™} and/or the like), and/or combinations thereof. For example, a hook-type coupler 175 may be coupled to the flange 132 and may be configured to couple to a loop-type coupler 173 coupled to or formed integrally with the carrier 165 or the substrate 110. The hook-and-loop type coupler (e.g., the hook-type coupler 175 and the loop-type coupler 173) may be continuous or discontinuous and may be situated such that they may be aligned with each other. In some embodiments, the loop-type coupler 173 may be formed integrally with the carrier 165.

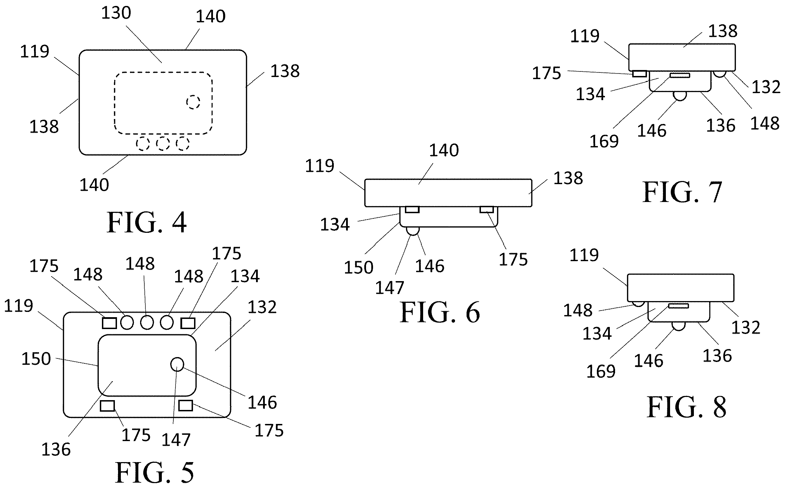

FIG. 3 shows an illustrative front side view of a portion of the control unit 119 coupled to the patch 101 of the AMU 200 of FIG. 2 in accordance with embodiments of the present system. The control unit 119 may be releasably coupled to the patch 101 by the coupler 167. The coupler 167 that may include the hook-and-loop type fastener and a snap-fit-type coupler including tabs 168 that may be configured to engage corresponding openings (e.g., see, openings 169, FIGs. 7 and 8) at the skirt 150.
The controller 119 may be releasably coupled to the sensors 114 to receive signals therefrom. The optical sensor 146 may include a facet surface 147 which may include a lens, filter, etc., that may extend beyond the lower surface 162 of the substrate 110 and/or carrier 165 so as to contact the skin on the patient during use.

FIG. 4 shows an illustrative top view of a portion of the control unit 119 of FIG. 2 in accordance with embodiments of the present system. The upper surface 130 may be situated between the side walls 140 and the end walls 138. FIG. 5 shows an illustrative bottom view of a portion of the control unit 119 of FIG. 2 in accordance with embodiments of the present system. Connectors 148 may extend from the flange 132. The optical sensor 146 may extend from the lower surface 136 and may include the facet surface 147. The hook-type couplers 175 may be coupled to the flange 132. At least one wall 134 may extend about the lower surface 136 to define at least a portion of the skirt 150.

FIG. 6 shows an illustrative rear side view of a portion of the control unit 119 of FIG. 2 in accordance with embodiments of the present system. The optical sensor 146 may extend from the lower surface 136 and include the facet surface 147. The at least one wall 134 may extend about the lower surface 136 to define at least a portion of the skirt 150. FIG. 7 shows an illustrative first end view of a portion of the control unit 119 of FIG. 2 in accordance with embodiments of the present system. FIG. 8 shows an illustrative second end view of a portion of the control unit 119 of FIG. 2 in accordance with embodiments of the present system. With reference to FIGs. 7 and 8, openings 169 may be configured to engage the tabs (e.g., see, 168, FIG. 2) to releasably couple the control unit 119 to the patch 101. The hook-type couplers 175 may be coupled to the flange 132 and may be configured to releasably couple the control unit 119 to the patch 101. The optical sensor 146 may extend from the lower surface 136 of the control unit 119. Connectors 148 may extend from the flange 132.

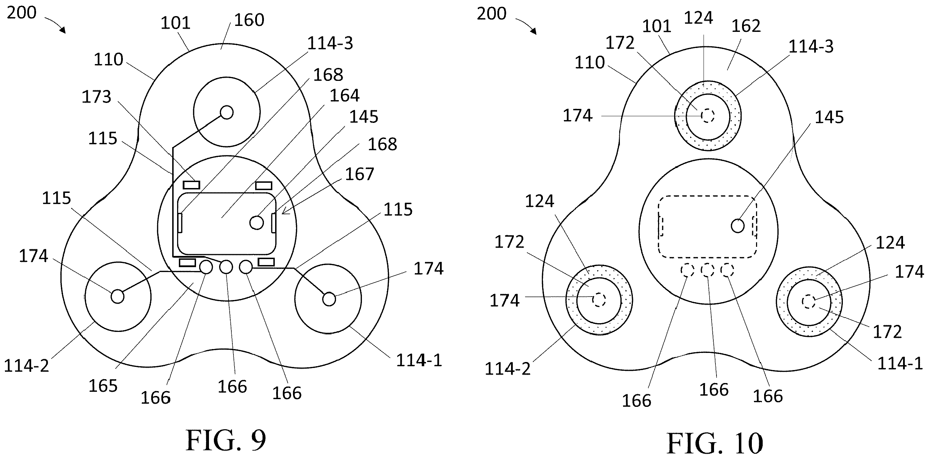

FIG. 9 shows an illustrative top planar view of the patch 101 of the system 200 of FIG. 2 in accordance with embodiments of the present system. FIG. 10 shows an illustrative top planar end view of the patch 101 of the system 200 of FIG. 2 in accordance with embodiments of the present system. With reference to FIGs. 9 and 10, the patch 101 may include the substrate 110, the carrier 165, and sensors 114 coupled to, or formed integrally with, the substrate 110. The sensors 114 may include electrodes 174 that may be coupled to connectors 166 via leads 115 to transfer surface EMG signals from a surface of the skin of the patient to the connectors 166. The connectors 166 may be coupled to, or formed integrally with, the carrier 165 and may be biased to assure coupling with the corresponding connectors (e.g., connectors 148, FIG. 2) of the control unit 119. When the control unit 119 is coupled to the patch 101, the connectors 148 may be coupled to the connectors 166 of the patch 101.

The cavity 164 of the carrier 165 may be configured to receive at least a portion of the control unit 119 and may include the opening 145 configured to receive the optical sensor (e.g., see, FIG. 3, the optical sensor 146) which may pass at least partially therethrough. The coupler 167 includes the tabs 168 configured to engage corresponding openings (e.g., see, FIGs. 7, 8, the openings 169) of the control unit 119 so as to secure the control unit 119 to the patch 101 (e.g., using a snap fit). The patch 101 may include the substrate 110, the carrier 165 and sensors 114 coupled to, or formed integrally with, the substrate 110. The sensors 114 may include a hydrogel 172 coupled to electrodes 174 that may be coupled to the corresponding connectors 166 via corresponding leads 115 of the leads 115 to transfer surface EMG signals from a surface of the skin of the patient to the connectors 166.

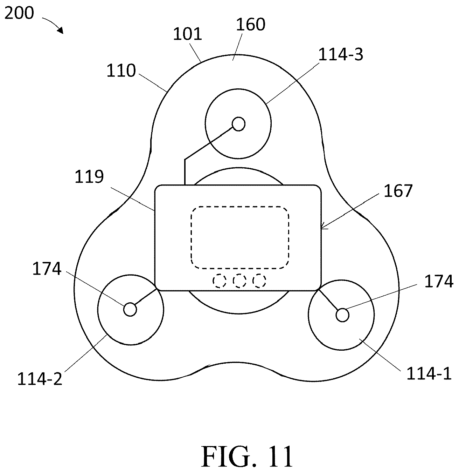

FIG. 11 shows an illustrative top view of a portion of the AMU 200 with the control unit 119 coupled to the patch 101 in accordance with embodiments of the present system. The control unit 119 may be releasably coupled to the patch 101 by the coupler 167. The sensors 114 may include the electrodes 174 that may be releasably coupled to the control unit 119.

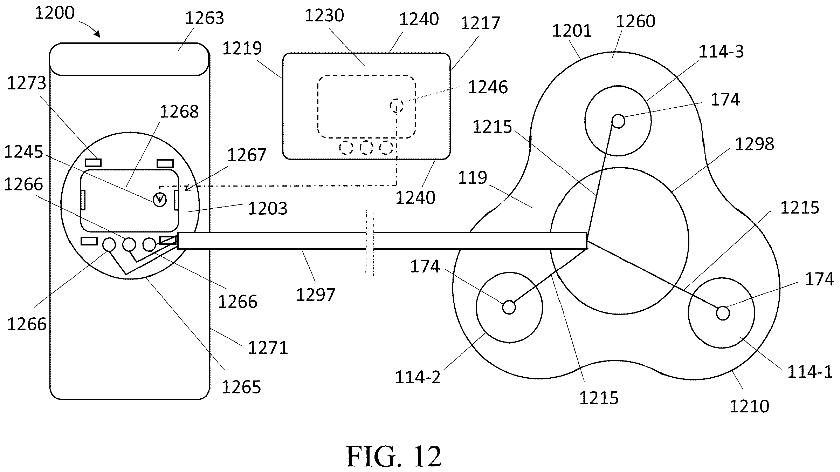

FIG. 12 shows an illustrative partially exploded top view of a system 1200 with a control unit 1219 mounted separately from a patch 1201 in accordance with embodiments of the present system. The control unit 1219 may be similar to the control unit 119 and may include a body 1217 having an upper surface 1230 and side walls 1240. The control unit 119 may be releasably coupled to a carrier 1265 such that a portion of the control unit 1219 may extend into a cavity 1268 of the carrier 1265 including connectors 1266 and a coupler 1267. The carrier 1265 may be similar to the carrier 165 of the embodiments shown and described with reference to FIG. 2. The carrier 1265 may be coupled to a band 1271 that may be couplable, for example, to an arm of a patient. The band 1271 may be formed from any suitable material such as an elastic material and may be configured to wrap around the arm of the patient to hold the control unit 1219 on the arm of the patient during use. The band 1271 may have ends or may be formed into a loop, through which, the arm of the patient may pass. A coupler such as a hook-and-loop (e.g., Velcro^{™}, etc.) type fastener 1263 may be provided to adjust the size of a loop of the band 1271 when desired so as to assure a comfortable fit about the arm of the patient during use. In yet other embodiments, the band 1271 may include an adhesive layer such that it may releasably couple to the skin of a patient during use. In yet other embodiments, the band 1271 may include a sliding buckle type adjuster. An opening 1245 may extend through the cavity 1268 and the band 1271 and may be configured to provide for an optical sensor 1246 (e.g., an SpO2 sensor) of the control unit 1219 to pass therethrough so as to contact the skin of the patient during use to sense information to determine, for example, peripheral oxygen saturation (SpO2). The patch 1201 may include sensors 114 having electrodes 174 that may couple to the connectors 1266 via leads 1215 that may be flexible and may be covered by a cover 1297. The leads 1215 may be coupled to an extension of the patch 101 or may pass through the cover 1297 (e.g., a sheath). The cover 1297 may be coupled to a reinforcing layer 1298 of the patch 1201 and/or the carrier 1265 so as to reduce a load on, at least, the leads 1215 during use.

The embodiments shown in FIG. 12, may be suitable for patients that like to sleep on their stomach in which case protrusions on the patch might be experienced as uncomfortable. By placing the CU elsewhere on the body (e.g., on the arm), the patch can remain very slim. A cable may connect the CU to the patch. The CU has to be placed in such a way that the integrated SpO2 sensor can perform its measurement on the arm of the patient and may extend through an opening in the band 1271 as discussed.

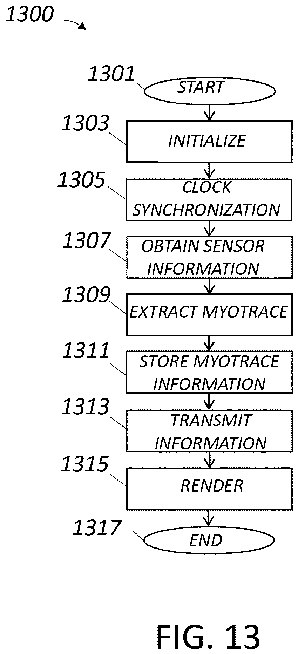

An operational process performed by an apnea measuring unit (AMU) communicating with a MS of the patient will now be described with reference to FIG. 13 which shows an illustrative functional flow diagram performed by a process 1300 in accordance with embodiments of the present system. The process 1300 may be performed using one or more processors, computers, controllers, etc., communicating over a network and may obtain information from, and/or store information to one or more memories which may be local and/or remote from each other. The process 1300 may include one of more of the following acts. In accordance with embodiments of the present system, the acts of process 1300 may be performed using a controller operating in accordance with embodiments of the present system. Further, one or more of these acts may be combined and/or separated into sub-acts, or performed serially or in parallel, as may be desired. Further, one or more of these acts may be skipped depending upon settings. For the sake of clarity, the process may be described with reference to a single AMU. However, without limitation, it should be understood that the process may employ a plurality of AMUs each of which may include a separate workflow such as a sub-workflow. In operation, the process 1300 may start during act 1301 and then proceed to act 1303.

During act 1303, the process may initialize the AMU by performing an initialization routine. During initialization, the process may load SSI and may set settings in accordance with the SSI. After completing act 1303, the process may continue to act 1305, wherein if communication is established between the AMU and the MS and/or the CSC depending upon system settings, a clock of the CU may be synchronized with the clock of the MS and/or the CSC. Accordingly, the CU and the MS and/or the CSC may perform a clock synchronization routine. This may assure that information and graphs transmitted from the CU to the MS and/or the CSC may be synchronized with respect to time. In some embodiments, the CU may be set to turn on/off at a certain time and/or may transmit data at a certain time (e.g., 8:00 am on Tuesday), depending upon system settings. In accordance with embodiments of the present systems, these acts (e.g., turn on/off, data transmission, etc.) may also be performed periodically (e.g., every 10 minutes).

After completing act 1305, the process may continue to act 1307 during which the process may cause sensor information to be obtained from one or more sensors of the AMU such as surface EMG signals (EMG) from the sensors (e.g., see FIG. 1, sensors 114), oxygen saturation signals from the optical sensor (e.g., see FIG. 2, optic sensor 146), acceleration signals from an acceleration sensor, etc.

After completing act 1307, the process may continue to act 1309 during which the process may cause the obtained sensor information to be analyzed to extract, for example, from the EMG signals, an effort of breathing (e.g., NRD such as Myotrace^{™}, respiratory muscle effort) measurement, heart rate, and limb movement (e.g., arm movement) data. During this act, the process may also determine surface oxygen saturation (SpO2) and acceleration data. The acceleration data may be determined from an acceleration sensor, such as a gyroscope, and may be employed to resolve movement artefacts from the NRD measurement.

For example, EMG signals may be recorded and thereafter processed to extract an approximation of the neuromuscular drive (NRD) (e.g., the "effort" exerted by the muscles to breathe). To go from the raw EMG signal on the electrode to a NRD calculation, several data processing steps may be taken: removal of heart rate (ECG), inhalation phase detection; artefact removal (e.g., caused by movement) and finally NRD calculation. From the physiological information, it may be difficult to identify motion artefacts, therefore an accelerometer may be included to provide an additional signal. While the SpO2 measurement may not be utilized for the NRD calculation, is may be used separately for sleep apnea detection (e.g., deoxygenation is a sign of poor perfusion). Normally SpO2 measurement may be performed with a finger clip, but a patch on the body may be utilized as a less intrusive method to measure SpO2 or may be used in addition to the finger clip to provide additional confidence in the measurement. In addition, the SpO2 measurement may be utilized in combination with other measurements to determine whether sleep apnea is present during the measurements. For example, if the measurements (e.g., one or more of the NRD determination, a heart rhythm determination and body movement signals) indicate that the breathing effort suddenly rises as well as the heart rate and the SpO2 lowers, an apnea event could be occurring. These episodes of sleep apnea may become apparent and detectable by the present system (e.g., one or more controller) in comparison to average values during one or more epochs, test periods, etc.

In accordance with embodiments of the present system, by the employment of the novel AMU to measure breathing effort, as measured by an effort of breathing measurement which reflects respiratory muscle effort (e.g., muscle activity of the body trying to breathe), heart rate and patient movement may be processed to determine if the patient has sleep apnea (e.g., determine whether the patient is experiencing sleep apnea and if present, a level of sleep apnea). Further, the heart rate and limb movement data may be stored separately (as discussed below) and may constitute a part of the measurements utilized to perform an apnea diagnosis.

After completing act 1309, the process may continue to act 1311 wherein the process may store the data generated during act 1309 in a memory of the system such as a memory of the CU. The data may be stored separately such that individual data (e.g., limb movement, heart rate, acceleration, SpO2, etc.) may be individually recalled for further processing which may be performed at a later time. After completing act 1311, the process may continue to act 1313 wherein the data may be transmitted (e.g., from its storage in the memory of the CU) to a desired recipient such as the MS and/or CSC. The transfer may be performed using any suitable transmission method such as wired and/or wireless, such as an optical transmission method. For example, in some embodiments, the CU may employ cellular communication methods (e.g., 5G service) to transfer the data to the CSC directly or via the MS depending upon system configuration and/or settings. In some embodiments, the data may be transferred upon pairing of devices (e.g., the CU and the MS) or upon a request of the clinician and/or patient. In some embodiments, the data may be transferred autonomously. For example, the CU may push data to the CSC at a certain time after it is received. This may provide for autonomous data transfer between the CU and the CSC with or without the MS. Accordingly, several methods of transmitting the data generated by the present process to a clinician (e.g., medical professional, etc.) for analysis may be used such as handing the CU in so that the data can be extracted with a physical connection, pairing the CU with an app such as a smartphone app, and/or by transmitting data autonomously through a wired/wireless network, such as a cellular network. The remote options may have preference to enable rapid diagnosis in remote locations.

After completing act 1313, the process may continue to act 1315, data generated by the system may be rendered on a rendering device of the system such as a display of the MS and/or the CSC. After completing act 1315, the process may continue to act 1317 where it may end.

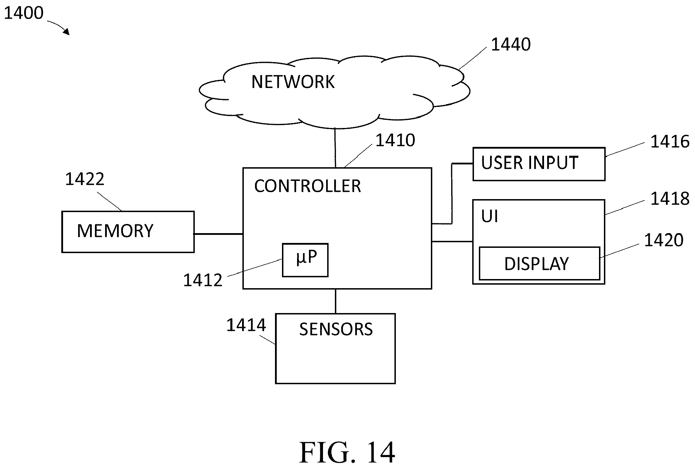

FIG. 14 shows an illustrative block diagram of a portion of a system 1400 (hereinafter system 1400 unless the context indicates otherwise) in accordance with embodiments of the present system. The system 1400 may include one or more of a controller 1410, sensors 1414, a user input interface 1416, a user interface (UI) 1418, a memory 1422 and a network 1440, one or more of which may be coupled to and/or communicate with each other using any communication method or methods such as wired and/or wireless communication methods. The system 1400 may be operative under the control of the controller 1410.

The controller 1410 may include one or more logic devices such as a microprocessor (µP) 1412 and may control the overall operation of the system 1400. It should be appreciated that in some embodiments the controller 1410 may include digital and/or analog control circuitry. It is envisioned that one or more portions of the system 1400, such as the controller 1410, may be operationally coupled to the memory 1422, the user interface (UI) 1418 including a rendering device such as a display 1420, the sensors 1414, the user input interface 1416 and the network 1440.

The memory 1422 may be any type of device for storing application data as well as other data related to the described operation such as application data, data generated by the system, operating parameters, etc., and/or combinations thereof. The application data, data generated by the system, operating parameters, etc., and/or combinations thereof, may be received by the controller 1410 for configuring (e.g., programming) the controller 1410 to perform operation acts in accordance with the present system. The controller 1410 so configured becomes a special purpose machine particularly suited for performing in accordance with embodiments of the present system.

The controller 1410 may render content, such as still or video information, as well as audio information on a rendering device of the system such as on the display 1420 and/or speaker of the UI 1418. This information may include information related to operating parameters, instructions, feedback, and/or other information related to an operation of the system or portions thereof such as SSI or portions thereof, clinical decision support, application data, data generated by the system, operating parameters, etc., and/or combinations thereof. Clinical decision support (CDS) may be employed to analyze data generated by the system and may generate determinations which may be stored in the system and/or may be rendered for the convenience of the patient and/or clinician depending upon settings in the SSI. The SSI may be system setting information that may be used by the system to set operational parameters and settings as may be set by the system (e.g., as default settings) and/or a user.

The sensors 1414 may be situated at one or more portions of the system and may sense related parameters, form corresponding sensor information and provide this sensor information to the controller 1410 for further processing. For example, the sensors 1414 may include sensors such as EMG sensors which may sense analog cardiac signals, may form corresponding sensor information (e.g., EMG signals, etc.) and may provide this information to the controller 1410 for further analysis. The sensors 1414 may be distributed throughout the system.

The user input interface 1416 may include a keyboard, a mouse, a trackball, touch sensitive sensor, or other device, such as a touch-sensitive display, which may be stand alone or part of a system, such as part of a laptop, a personal digital assistant (PDA), a mobile phone (e.g., a smart phone), a smart watch, an e-reader, a monitor, a smart or dumb terminal, the AMU and/or other device for communicating with the controller 1410 via any operable link such as a wired and/or wireless communication link. The user input interface 1416 may be operable for interacting with the controller 1410 including enabling interaction within a UI 1418 as described herein. Clearly the controller 1410, the sensors 1414, the user input interface 1416, the user interface (UI) 1418, the memory 1422 and the network 1440 may all or partly be a portion of a computer system or other device. The UI 1418 may be operative to provide audio/visual feedback to the user of the present system and may inform the operator of operating parameters, operating states, etc.

The methods of the present system are particularly suited to be carried out by a computer software program, such program containing modules corresponding to one or more of the individual steps or acts described and/or envisioned by the present system. Such program may of course be embodied in a computer-readable medium, such as an integrated chip, a peripheral device and/or memory, such as the memory 1422 or other memory coupled to the controller 1410.

The program and/or program portions contained in the memory 1422 may configure the controller 1410 to implement the methods, operational acts, and/or functions disclosed herein. The memories may be distributed, for example between the clients and/or servers, or local, and the controller 1410, where additional processors may be provided, may also be distributed or may be singular. The memories may be implemented as electrical, magnetic or optical memory, or any combination of these or other types of storage devices. Moreover, the term "memory" should be construed broadly enough to encompass any information able to be read from or written to an address in an addressable space accessible by the µP 1412 of the controller 1410. With this definition, information accessible through a network such as the network 1440 is still within the memory, for instance, because the processor 1412 may retrieve the information from the network 1440 for operation in accordance with embodiments of the present system.

The controller 1410 is operable for providing control signals and/or performing operations in response to input signals from the user input device 1416 as well as in response to other devices of a network, such as the sensors 1414, and executing instructions stored in the memory 1422. The µP 1412 may include one or more of a microprocessor, an application-specific and/or general-use integrated circuit(s), a logic device, etc. Further, the µP 1412 may be a dedicated processor for performing in accordance with the present system and/or may be a general-purpose processor wherein only one of many functions operates for performing in accordance with the present system. The µP 1412 may operate utilizing a program portion, multiple program segments, and/or may be a hardware device utilizing a dedicated or multi-purpose integrated circuit.

Further variations of the present system would readily occur to a person of ordinary skill in the art and are encompassed by the following claims.

An important aspect of embodiments of the present system is that they may make use of a novel measurement (e.g., the breathing effort) as an alternative to the current measurements of breathing pattern and airflow. Rather than instances of the breathing pattern/airflow being disturbed, the muscle activity of the body trying to breathe is used as a measure for detecting apnea present in a patient. For example, when a certain muscle effort does not provide sufficient ventilation (i.e., CO2 removal), the lungs start exerting more effort. For example, in the case of a collapsed part of the airway, this additional effort will recruit other areas of the lung more, keeping the net flow and thus ventilation the same. It is only when there is no more additional lung to recruit that the flow will drop and thereby the condition may only then be previously measured. In accordance with the present system, by measuring the respiratory effort, this deteriorating trend may be detected earlier. Besides being less invasive than conventional systems and methods, this could have added value in early deterioration detection, e.g., of a partially collapsed airway that may be detected by embodiments of the present system, well before the deterioration starts to influence the airflow, giving a more nuanced view than heretofore was available by prior systems.

In prior systems, muscle movement such as limb movement (e.g., the arms), heart rhythm, etc. may contribute to noise signals. Prior Apnea measurements are sensitive to the noise from muscle movements like the arms and the beating of the heart. Accordingly, in prior systems, the measurements during the noise are filtered out. Accordingly, in these prior systems, the apnea measurement is only performed when the patient is lying still, which is assessed by an accelerometer. However, in embodiments of the present system, signals that occur during noise (e.g., heart rhythm, limb movement, etc.) may be used and captured separately, as they constitute a part of the measurements that may be utilized to perform an apnea diagnosis. For example, next to collapsing airways, another cause of disturbed sleep is periodic limb movements (PLMS). This is also monitored for in some sleep diagnosis tests. In accordance with the present system, an accelerometer in the patch may be used to filter out artefacts, however, this information may also simultaneously be used to determine if PLMS is occurring. The acceleration information may also be utilized regarding heart rate, which is part of the electrode signal, and is filtered out, but can also be exported for analysis. Heart rate is one or the symptoms of sleep apnea (tachycardia) and therefore, the determined heart rate may be utilized in the determination for sleep apnea.

In this way, several electrodes for limb movement, a breathing monitor belt and nasal cannula of conventional systems may be replaced with one patch, significantly increasing patient comfort.

It is envisioned that embodiments of the present system may be operative without lengthy cables or hoses of conventional systems, or may have a single slim cable or lead, thus, reducing or entirely eliminating the discomfort of the cabling of conventional systems and methods. Further, embodiments of the present system replace several measurements (e.g., chest band, nasal cannula, limb movement electrodes of conventional systems) with one or two electrical measurements such as surface EMG on the upper chest of the user using a single type of sensor and integrates this single type of sensor (e.g., optical) that detects peripheral oxygen saturation (SpO2) and an accelerometer in a single adhesive electrode patch which enhances user convenience. Systems in accordance with the present system further reduces, cost, setup time, setup difficulty and discomfort to the patient. Accordingly, user setup is enhanced and discomfort during use is reduced over conventional systems and methods.

Accordingly, embodiments of the present system may improve the comfort and performance of the home sleep tests for sleep apnea. In accordance with embodiments of the present system, testing performance is improved by introducing a new measurement modality that gives insight into the breathing effort (also known as work of breathing), rather than use of the effectuated airflow as in conventional systems. In this way, the present system gives a more nuanced view on the airway effects leading to apnea. In accordance with the present system, the work of breathing shows the functionality of the operational engine behind the respiratory system (also called neural respiratory drive), instead of only showing the result of that (such as e.g., airflow, respiration rate, etc.) as is done in prior systems. Surprisingly, in the present system, the work of breathing and neural respiratory drive has been found to increase as a result of obstructive sleep apnea (OSA). By improving the performance, ease, and comfort of home apnea tests with the present system, fewer patients may need to be referred to spend the night in a sleep disorder center, with its associated disruption to sleep, inconvenience, cost, and discomfort.

Finally, the above discussion is intended to be merely illustrative of the present system and should not be construed as limiting the appended claims to any particular embodiment or group of embodiments. Thus, while the present system has been described with reference to exemplary embodiments, it should also be appreciated that numerous modifications and alternative embodiments may be devised by those having ordinary skill in the art including using features that are described with regard to a given embodiment with other envisioned embodiments without departing from the broader and intended spirit and scope of the present system as set forth in the claims that follow. In addition, any section headings included herein are intended to facilitate a review but are not intended to limit the scope of the present system. In addition, the specification and drawings are to be regarded in an illustrative manner and are not intended to limit the scope of the appended claims.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function;
e) any of the disclosed elements may be comprised of hardware portions (e.g., including discrete and integrated electronic circuitry), software portions (e.g., computer programming) and any combination thereof;
f) hardware portions may be comprised of one or both of analog and digital portions;
g) any of the disclosed devices, features and/or portions thereof may be combined together or separated into further portions unless specifically stated otherwise;
h) no specific sequence of acts or steps is intended to be required unless specifically indicated; and
i) the term "plurality of' an element includes two or more of the claimed element and does not imply any particular range of number of elements; that is, a plurality of elements may be as few as two elements and may include an immeasurable number of elements.

## Claims

1. A mobile electromyography (EMG) device (100), comprising:
a control unit (102) having at least one controller (120), a body (117) having opposed sides (130, 136), an optical sensor (146) extending from one of the opposed sides (130, 136), and a plurality of connectors (148) coupled to the at least one controller (120);
a patch (101) having a carrier (165) with a cavity (164) configured to receive at least part of the body (117), and an opening (145) extending through the patch (101) and configured to receive at least a portion of the optical sensor (146), the patch (101) further having an adhesive backed substrate (124) configured to releasably couple to the skin of a user, and a plurality of sensors (114) situated apart from each other and configured to sense surface EMG signals from the skin of the user; and
a coupler (167) configured to releasably couple the body (117) of the control unit (102) to the patch (101),
wherein the at least one controller (120) is configured to receive the sensed surface EMG signals from the plurality of sensors (114) and determine a Neural Respiratory Drive (NRD) measurement based thereon.

2. The mobile EMG device (100) of claim 1, wherein the at least one controller (120) is further configured to analyze the EMG signals from the plurality of sensors (114) to determine heart rhythm and body movement signals.

3. The mobile EMG device (100) of claim 2, wherein the at least one controller (120) is further configured to determine whether apnea exists based upon the NRD measurement and the heart rhythm and body movement signals.

4. The mobile EMG device (100) of claim 3, wherein the at least one controller (120) is further configured to transmit the NRD measurement and the heart rhythm and body movement signals to a mobile station (MS) (104) for further analysis.

5. The mobile EMG device (100) of claim 1, wherein the coupler (167) comprises at least one of a hook-and-loop fastener and a snap-fit fastener.

6. The EMG device (100) of claim 1, wherein the body (117) further comprises an accelerometer (118) situated in a cavity thereof and configured to detect acceleration of the body and output a corresponding signal.

7. The EMG device (100) of claim 6, wherein the at least one controller (120) is further configured to receive the output signal of the accelerometer (118) and form corresponding acceleration information.

8. The EMG device (100) of claim 7, wherein the at least one controller (120) is further configured to process the acceleration information to resolve movement artefacts in the EMG signals.

9. The EMG device (100) of claim 1, wherein the at least one controller (120) is further configured to receive an output signal from the optical sensor and determine peripheral oxygen saturation (SpO2) based thereon.

10. A mobile electromyography (EMG) device (100, 1200), comprising:
a control unit (119, 1219) having at least one controller (120, 1219), a body (117, 1217) having opposed sides (130, 136, 1230), an optical sensor (146, 1246) extending from one of the opposed sides (130, 136, 1230), and a plurality of connectors (148) coupled to the at least one controller (120, 1219);
a carrier (165, 1265) with a cavity (164, 1268) configured to receive at least part of the body (117, 1217), and an opening (145, 1245) extending through the carrier (165, 1265) and configured to receive at least a portion of the optical sensor (146, 1246);
a patch (101, 1201) having an adhesive backed substrate (124) configured to releasably couple to the skin of a user, and a plurality of EMG sensors (114) situated apart from each other and configured to sense surface electromyography (sEMG) signals from the skin of the user;
a coupler (167, 1267) configured to releasably couple the body (117, 1217) of the control unit (119, 1219) to the carrier (165, 1265) and being coupled to the patch (101, 1201); and
a flexible strip (1271) coupled to the carrier (165, 1265) and configured to couple to the user,
wherein the at least one controller (120, 1219) is configured to receive the sEMG signals from the plurality of sensors (114) and determine a Neural Respiratory Drive (NRD) measurement based thereon.

11. The mobile EMG device (100, 1200) of claim 10, wherein the at least one controller (120, 1219) is further configured to receive an output signal from the optical sensor (146, 1246) and determine peripheral oxygen saturation (SpO2) based thereon.

12. The mobile EMG device (100, 1200) of claim 10, wherein the flexible strip (1271) comprises an adjustable loop.

13. An electromyography (EMG) electrode patch (100, 1200), comprising:
a carrier (165, 1265) with a cavity (164, 1268) configured to receive at least part of a body (117, 1217), and an opening (145, 1245) extending through the carrier (165, 1265) and configured to receive at least a portion of an optical sensor (146, 1246), the carrier (165, 1265) further having a plurality of sensors (114) situated apart from each other and configured to sense surface EMG signals from the skin of the user; and
a coupler (1263) configured to releasably couple the body (117, 1217) of a control unit (119, 1219) to the carrier (165, 1265).

14. The EMG patch (100, 1200) of claim 13, further comprising a plurality of contacts (165, 1265) with each contact coupled to a corresponding sensor (114-1, 114-2, 114-3) of the plurality of sensors (114) and configured to couple to a corresponding biased contact of a plurality of biased contacts (166, 1266) coupled to the body (117, 1217) of the control unit (119, 1219).

15. The EMG patch (100, 1200) of claim 13, further comprising the control unit (119, 1219) having at least one controller (120, 1219), the control unit (119, 1219) having the body (117, 1217) and the optical sensor (146, 1246), the body (117, 1217) having opposed sides (130, 136, 1230), the optical sensor (146, 1246) extending from one of the opposed sides (130, 136, 1230).
